# EUROPÄISCHE PATENTSCHRIFT

(11) **EP 1 149 065 B1**
(45) Veröffentlichungstag und Bekanntmachung des Hinweises auf die Patenterteilung: **12.01.2005**
(21) Anmeldenummer: 00904982.6
(22) Anmeldetag: 29.01.2000
(51) Int. Cl.: C07C 67/54, C07C 51/48, C07C 69/708, C07C 59/125

(54) **VERFAHREN ZUR HERSTELLUNG VON ESTERN**
METHOD FOR THE PRODUCTION OF ESTERS
PROCEDE DE PREPARATION D'ESTERS

(30) Priorität: 03.02.1999 DE 19904207
(43) Veröffentlichungstag der Anmeldung: 31.10.2001
(73) Patentinhaber: BASF AKTIENGESELLSCHAFT, 67056 Ludwigshafen (DE)
(72) Erfinder: DITRICH, Klaus, D-67161 Gönnheim (DE); BLOCK, Ulrich, D-67159 Friedelsheim (DE)
(86) Internationale Anmeldenummer: PCT/EP2000/000702
(87) Internationale Veröffentlichungsnummer: WO 2000/046177

(56) Entgegenhaltungen:
- EP-A- 0 361 839
- DATABASE WPI Section Ch, Week 199122 Derwent Publications Ltd., London, GB; Class E14, AN 1991-161783 XP002139633 & RO 100 193 A (INST POLITEHN TIMIS), 30. Juli 1990 (1990-07-30)
- DATABASE WPI Section Ch, Week 199244 Derwent Publications Ltd., London, GB; Class A41, AN 1992-363082 XP002139634 & JP 04 266845 A (JAPAN SYNTHETIC RUBBER CO LTD), 22. September 1992 (1992-09-22)

## Beschreibung

Die vorliegende Erfindung betrifft ein neues Verfahren zur Herstellung von Estern der allgemeinen Formel I sowie ein Verfahren zur Extraktion von Verbindungen der Formel II.

Ester der allgemeinen Formel I sind wertvolle Zwischenprodukte für die Racematspaltung von Aminen durch Enzym-katalysierte Umsetzung mit diesen Estern.

So beschreibt Kitaguchi et al. (J. Amer. Chem. Soc. 111, 3094-3095, 1989) beispielsweise die Racematspaltung von Aminen mit Trifluorethylbutyrat unter Subtilisin-Katalyse. Von Gotor et al. (J. Chem. Soc. Chem. Commun. 957-958, 1988) wird die enantioselektive Acylierung von 2-Amino-butan-1-ol mit Ethylacetat unter Katalyse von Schweinepankreas-Lipase (PPL) beschrieben.

Quiros et al. (Tetrahedron: Asymmetry 4, 1105-1112, 1993) beschreiben die Lipase-katalysierte Synthese von optisch aktiven Amiden aus racemischen α-Halogen-substituierten Propionsäureethylestern und primären Aminen.

In US 5,057,607 wird ein Verfahren zur stereoselektiven Acylierung von primären Aminen mit Estern, die in Nachbarschaft zum Carbonylkohlenstoff ein Sauerstoffatom tragen, zur Synthese von β-Lactamen beschrieben.

WO 95/08636 beschreibt ein Verfahren zur Racematspaltung von primären und sekundären Aminen in Gegenwart eines Esters mit einer Hydrolase.

Bei der in WO 95/08636 beschriebenen Enzym-katalysierten kinetischen Racematspaltung von Aminen wird ein Enantiomer in das Amid umgewandelt. Für diese enzymatische Acylierungreaktion werden bevorzugt Ester verwendet, die in α-Position zum Carbonylkohlenstoff ein Sauerstoffatom tragen wie beispielsweise Methoxyessigsäureester. Aus dem in dieser Reaktion entstehenden Amid wird das freie Amin durch Spaltung mit einer Base gewonnen. Dabei fällt neben dem Amin die Säure beispielsweise die Methoxyessigsäure in Form ihrer Salze in wäßriger Lösung an. Für die Wirtschaftlichkeit des Verfahrens ist es wichtig, diese Säure wieder für die Acylierungsreaktion verfügbar zu machen, das heißt sie wieder in ihren Ester zu überführen, der dann erneut in der enzymatischen Acylierung verwendet werden kann.

Üblicherweise werden derartige Ester aus den Säuren in Gegenwart eines Alkohols und einer Mineralsäure als Katalysator gebildet. Diese Esterbildung erfolgt jedoch nur bis zu einem Gleichgewicht.

In Fällen, in denen die Siedepunkte der Säuren, Alkohole und Ester über denen des Wassers liegen, kann das Gleichgewicht durch Entfernen des Wassers über eine Destillation leicht verschoben werden. Liegen die Siedepunkte unter dem des Wassers, kann diese Methode nicht angewandt werden.

In der Literatur werden eine Reihen von Methoden beschrieben, in denen versucht wurde das Problem der niedrigeren Siedepunkte der Ester und Alkohole in den Griff zu bekommen. In DE 195 39 293 wird so beispielsweise ein Verfahren zur Herstellung von Cyanessigsäurealkylestern beschrieben, in dem das Wasser über eine azeotrope Destillation entfernt wird. Von Nachteil bei diesem Verfahren ist, daß ein vollständiger Umsatz unter den beschriebenen Bedingungen nicht erreicht werden kann.

CH 527 156 beschreibt ebenfalls ein Verfahren zur Herstellung von Estern hochsiedender Carbonsäuren. In diesem Verfahren wird das Gleichgewicht und damit die Reaktion durch einen großen Alkoholüberschuß beeinflußt. Nachteilig ist, daß der über die Destillation entfernte Alkohol ständig während der Reaktion nachgeliefert werden muß.

Von Nachteil bei den oben beschriebenen azeotropen Destillationen ist, daß das Reaktionswasser nicht vollständig entfernt werden kann und daß dadurch ein vollständiger Umsatz nicht möglich ist.

In EP-B-0 361 839 wird ein Verfahren beschrieben, das diesen Nachteil nicht hat. EP-B-0 361 839 beansprucht ein Verfahren zur Dehydratisierung von Substanzen und Mischungen, durchgeführt durch kontinuierliche azeotrope Destillation mit einem organischen Lösungsmittel, das mit Wasser eine praktisch unmischbare azeotrope Mischung mit minimalen Siedepunkt bildet, wobei das Kondensationsdestillat auf zumindest eine Temperatur gekühlt wird, bei der das Kondensat mit einem gegebenen Wassergehalt oder die organische Phase des Kondensates mit Wasser übersättigt wird, wodurch eine weitere Wassertrennung ermöglicht wird. Dieses Verfahren ist leider nicht breit anwendbar und erfordert als weiteren Verfahrensschritt eine Kühlung des Kondensats.

In US 5,202,463 wird ein mehrstufiges Verfahren zur Abtrennung des Wassers, das bei der Veresterung entsteht, beschrieben. Dieses Verfahren erfordert daher einen hohen apperativen Aufwand.

Da keines der bekannten Verfahren eine möglichst vollständige Umsetzung zu den Estern ermöglicht und leicht und einfach durchführbar ist, bestand daher die Aufgabe, ein entsprechendes Verfahren zu entwickeln, das die Nachteile der oben genannten Verfahren nicht aufweist und eine einfache, kostengünstige Herstellung von Estern aus den in wäßriger Lösung enthaltenden Säuren ermöglicht.

Diese Aufgabe wurde durch ein Verfahren zur Herstellung von Estern der allgemeinen Formel I, 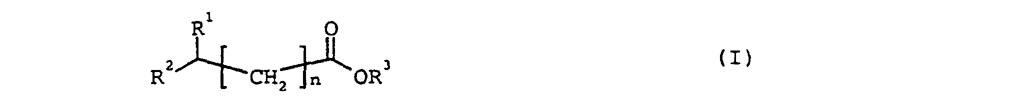 aus in wäßrigen Lösungen enthaltenen Verbindungen der allgemeinen Formel II, 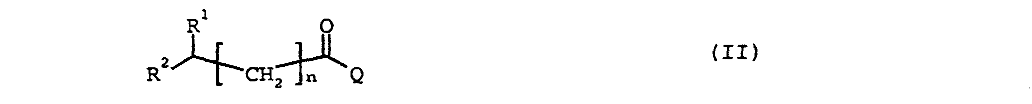 dadurch gekennzeichnet, daß
a) die Verbindungen der allgemeinen Formel II direkt oder nach Freisetzung aus ihren Salzen in Gegenwart eines C₁-C₈-Alkohols und einem mit Wasser nicht mischbaren Lösungsmittel extrahiert werden und
b) anschließend in Gegenwart eines Katalysators und eines Schleppmittels unter Bedingungen einer azeotropen Destillation mit dem C₁-C₈-Alkohol verestert werden, gelöst,
wobei die Verfahrensschritte (a) und (b) zeitlich und räumlich getrennt oder aber in einer aufeinanderfolgenden kontinuierlichen oder diskontinuierlichen Sequenz durchgeführt werden können und wobei die Variablen und Substituenten in den Formeln I und II folgende Bedeutung haben:
- R¹ =: F, Cl, -OH, -OC₁-C₁₀-Alkyl,
- R² =: H, C₁-C₁₀-Alkyl
- R³ =: C₁-C₈-Alkyl,
- Q =: -OH, -O⁻ K⁺, wobei K⁺ ein Alkali- oder Erdalkalikation oder ein Amin ist,
- n =: 0,1 oder 2, bevorzugt 0 oder 1, besonders bevorzugt 0.

R¹ bezeichnet in den Verbindungen der Formeln I und II Fluor, Chlor, Hydroxy-, substituiertes oder unsubstituiertes, verzweigtes oder unverzweigtes -OC₁-C₁₀-Alkyl.

Als -O-Alkylreste seien substituierte oder unsubstituierte verzweigte oder unverzweigte -O-C₁-C₁₀-Alkylketten, in denen die Bindung der Ketten über ein Sauerstoffatom an das Grundgerüst der Formeln erfolgt und in denen die Alkylreste beispielsweise folgende Bedeutung haben Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl. Als bevorzugte Reste seien Methyl-, Ethyl- oder n-Propyl- genannt.

R² bezeichnet in den Verbindungen der Formeln I und II Wasserstoff oder substituiertes oder unsubstituiertes, verzweigtes oder unverzweigtes C₁-C₁₀-Alkyl-.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Als bevorzugte Reste seien Wasserstoff, Methyl-, Ethyl- oder n-Propyl- genannt. Besonders bevorzugt ist Wasserstoff oder der Methylrest.

R³ bezeichnet in den Verbindungen der Formeln I substituiertes oder unsubstituiertes, verzweigtes oder unverzweigtes C₁-C₈-Alkyl.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Als bevorzugte Reste seien Ethyl-, n-Propyl-, iso-Propyl-, Butyl-, Hexyl- oder Octyl- genannt. Besonders bevorzugt ist der iso-Propylrest.

Als Substituenten der genannten Reste R¹, R² und R³ kommen Substituenten wie F, Cl, Br, CN, O-CH₃ und/oder O-Phenyl in Frage.

Die Variable Q in den Verbindungen der allgemeinen Formel I bedeutet Hydroxy oder -O⁻ K⁺, wobei K⁺ ein Alkali- oder Erdalkalikation oder ein Amin ist. Als Alkali- oder Erdalkalikationen seien beispielhaft die Kationen des Lithium, Natrium, Kalium oder Calcium genannt. Bevorzugt sind die des Natrium oder Kalium. Unter Amin sind beispielsweise organische Amine oder NH₃ zu verstehen.

Im erfindungsgemäßen Verfahren werden die für die Veresterung zu verwendenden Säuren zunächst in der wäßrigen Lösung falls erforderlich aus ihren Salzen freigesetzt. Hierzu eignen sich alle Säuren deren pKₛ-Wert niedriger sind als der pKₛ-Wert der Verbindungen der allgemeinen Formel II (Q = OH). Im Falle von Methoxyessigsäure sind dies alle Säuren, die einen pKₛ-Wert kleiner ca. 2 aufweisen und damit die Methoxyessigsäure aus ihrem Salz beispielsweise ihrem Natriumsalz freisetzen können. Geeignete Säuren sind beispielsweise Mineralsäuren wie Schwefelsäure, Salzsäure, Salpetersäure, Phosphorsäure oder organische Säuren wie Ameisensäure, Oxalsäure, Chloressigsäure, Fluoressigsäure, Cyanessigsäure, Benzolsulfonsäure, o- oder p-Toluolsulfonsäure, Methansulfonsäure, Trifluormethansulfonsäure oder Triflouressigsäure. Die Verwendung von Schwefelsäure oder p-Toluolsulfonsäure ist bevorzugt, wobei insbesondere die Verwendung von Schwefelsäure aus Kostengründen bevorzugt ist.

Die Freisetzung der Säure kann auch über einen sauren Ionentauscher erfolgen, der einen pKₛ-Wert hat, der in der Lage ist die Säure aus ihrem Salz zu befreien.

Nach Freisetzung der Säuren der allgemeinen Formel II (Q = OH) aus ihren Salzen werden diese mit einem C₁-C₈-Alkohol und einem apolaren organischen mit Wasser nicht mischbaren Lösungsmittel extrahiert. Derartige Lösungsmittel sind in einer Reihe von Stoffklassen zu finden beispielsweise unter den gradkettigen oder verzweigtkettigen gesättigten oder ungesättigten Kohlenwasserstoffen wie n-Heptan, n-Hepten, n-Ocatan oder n-Octen und deren verzweigten Isomeren, den gesättigten cyclischen Kohlenwasserstoffen die Cyclohexan oder Cycloheptan, offenkettige oder cyclische, gesättigte oder ungesättigte Ether oder Thioether oder aromatischen Verbindungen wie Benzol, Toluol oder Xylol. Bevorzugt sind aromatische Verbindungen besonders bevorzugt ist Toluol.

Vorteilhaft wird die wäßrige Lösung der freigesetzten Säure mit etwa 5 % Wasser verdünnt, um zu verhindern, daß Salze der zur Freisetzung verwandten Säure, beispielsweise der Schwefelsäure, ausfallen.

Die Menge des Lösungsmittelgemisches für die Extraktion ist nicht kritisch. Das Lösungsmittel kann in einem vielfachen Überschuß für die Extraktion verwendet werden. Vorteilhafterweise wird es bezogen auf die wäßrige Lösung der Säure in nahezu gleichen Volumenmengen verwendet, das heißt in einem Verhältnis von 1 : 1 bzw. 1 : 1,5 bezogen auf die wäßrige Lösung der Säure.

Für die Extraktionsausbeute im erfindungsgemäßen Verfahren ist es von entscheidender Bedeutung, daß die Extraktion in Gegenwart eines C₁-C₈-Alkohols durchgeführt wird. Ohne den Alkohol beträgt die Extraktionsausbeute nur wenige Prozent. Geeignete Alkohole sind Methanol, Ethanol, Propanol, iso-Propanol, Butanol und seine Isomere sowie Pentanol, Hexanol, Heptanol und Octanol und deren verzweigte Isomere. Bevorzugt sind Ethanol, Propanol, iso-Propanol, Butanol, Hexanol oder Octanol. Besonders bevorzugt ist iso-Propanol. Prinzipiell sind auch Ketone zur Verbesserung der Extraktion geeignet. Durch die Zugabe des Alkohols lassen sich die Säuren mit einer Ausbeute von mindestens 85 %, bevorzugt von mindestens 90 % besonders bevorzugt von mindestens 95 % extrahieren.

Bei der Extraktion wird vorteilhaft ein Verhältnis von Alkohol zu organischem Lösungsmittel von 1 : 2 bis 5 : 1 eingestellt. Bevorzugt wird ein Verhältnis von Alkohol zu organischem Lösungsmittel von 1 : 1 bis 3 : 1 eingestellt. Besonders bevorzugt wird ein 3 : 1 Gemisch verwendet. Ein weiteres Co-Solvens wie Cyclohexan ist von Nachteil, da sich drei Phasen ausbilden.

Die Alkohole können im erfindungsgemäßen Verfahren auch gleichzeitig als Lösungsmittel verwendet werden.

Zur Verbesserung der Extraktionsergebnisse können gegebenenfalls Salze zugesetzt werden.

Verfahrensschritt (a) [= Extraktion] kann im erfindungsgemäßen Verfahren prinzipiell bei jeder Temperatur durchgeführt werden, bei der der Alkohol und/oder das Lösungsmittel nicht nennenswert flüchtig ist und die Extraktion möglichst rasch erfolgen kann. Vorteilhaft wird die Extraktion bei einer Temperatur von 0°C bis 70°C durchgeführt. Bevorzugt wird die Extraktion bei einer Temperatur von 20°C bis 50°C durchgeführt.

Für die Extraktion kann entweder die freigesetzte Säure vorgelegt werden oder der Alkohol und/oder das Lösungsmittel und die anderen jeweils anderen Komponenten zugegeben werden. Vorteilhaft werden alle Komponenten gleichzeitig zusammengegeben. Die Extraktion kann batch-weise, semi-kontinuierlich (mehrmalige Zugabe eines oder mehrer Komponenten) oder kontinuierlich durchgeführt werden. Vorteilhaft wird die Extraktion kontinuierlich durchgeführt.

Die für das erfindungsgemäße Verfahren bevorzugt geeigneten Säuren führen zu Estern, die in der Säurekomponente des Esters ein Sauerstoffatom oder Fluoratom in Nachbarschaft zum Carbonylkohlenstoff tragen.

Unter Nachbarschaft zum Carbonylkohlenstoff ist die Bindung des Heteroatoms an ein Kohlenstoffatom in alpha-, beta- oder gamma-Position zum Carbonylkohlenstoff zu verstehen. Bevorzugt sind solche Säurekomponenten des Esters, bei denen das Heteroatom an das C-alpha Atom gebunden ist. Als Heteroatom ist Sauerstoff bevorzugt.

Das Heteroatom kann im Falle des Sauerstoffs gegebenenfalls mit weiteren Gruppen, z.B. Alkylgruppen, verknüpft sein. Dies führt zu Ethern.

Unter diesen sind die C₁-₈-Alkylester der C₁-₄-Alkoxyessigsäuren. wie der Methoxyessigsäureethylester, bevorzugt.

Als Katalysatoren der Esterbildung aus den extrahierten Säuren und den C₁-C₈-Alkoholen, die bei der Extraktion vorteilhaft verwandt wurden, sind prinzipiell alle Katalysatoren geeignet, die eine Esterbildung katalysieren können wie Carbonate, Säuren oder saure Ionenaustauscher. Vorteilhaft geeignete Säuren sind starke bis mittelstarke anorganische oder organische Säuren, wie Schwefelsäure, Salzsäure, Phosphorsäure, Oxalsäure, Ameisensäure, Benzosulfonsäure, Toluolsulfonsäure, Methansulfonsäure, Trifluoressigsäure, Fluoressigsäure, Mono-, Di- oder Trichloressigsäure oder Trifluormethansulfonsäure. Bevorzugt sind aus Kostengründen Schwefelsäure und p-Toluolsulfonsäure, besonders bevorzugt Schwefelsäure. Geeignete Ionenaustauscher sind beispielsweise die verschiedenen Amberlyte wie Amberlyst W38.

In der Veresterung werden vorteilhaft dieselben Alkohole verwendet, die auch schon für die Extraktion verwendet wurden. Es können aber auch andere Alkohole oder Alkoholgemische verwendet werden.

Für das erfindungsgemäße Verfahren sind im Verfahrensschritt (b) [Veresterung] prinzipiell alle organischen Lösungsmittel als Schleppmittel geeignet, die mit Wasser nicht mischbar sind und deren Siedepunkt als Azeotrop mit dem Alkohol und dem Wasser um mindestens 8°C, bevorzugt um mindestens 10°C, besonders bevorzugt um mindestens 14°C niedriger ist als das Azeotrop aus dem Alkohol und dem Wasser. Vorteilhaft geeignete Lösungsmittel sind beispielsweise Cyclohexan, Cyclopentan, n-Hexan, n-Heptan, Methylcyclohexan.
Bevorzugt wird als Schleppmittel Cyclohexan verwendet.

Für die Veresterung werden mindestens 1 Gew-% Schleppmittel bezogen auf das Gesamtvolumen der Reaktion verwendet. Es kann auch in einem großen Überschuß verwendet werden. Vorteilhaft werden 5 bis 50 Gew-% Schleppmittel, besonders bevorzugt 5 bis 20 Gew-%, ganz besonders bevorzugt 5 bis 10 Gew-% Schleppmittel für die Veresterung verwendet.

Die Veresterung wird vorteilhaft bei einer Temperatur von 50°C bis 100°C, besonders bevorzugt von 60°C bis 90°C durchgeführt.

Das erfindungsgemäße Verfahren kann bei Normaldruck oder bei erhöhtem oder verringertem Druck durchgeführt werden. Dabei können die Verfahrensschritte (a) und (b) bei gleichen oder unterschiedlichen Drücken durchgeführt werden. Bevorzugt wird das Verfahren bei Normaldruck oder erhöhtem Druck durchgeführt.

Durch die vorteilhaften Schleppmittel kann der Wassergehalt in der Veresterungsreaktion unter 1 % bevorzugt unter 0,5 % besonders bevorzugt unter 0,1 % gedrückt werden. Der Wassergehalt wurde nach der Karl-Fischer-Titration bestimmt. Der niedrige Wassergehalt ermöglicht eine Ausbeute an Ester von mindestens 90 %, bevorzugt von mindestens 95 %, besonders bevorzugt von mindestens 97 %, ganz besonders bevorzugt von mindestens 99 %.

Durch die Benutzung der Schleppmittel wird außerdem die Reaktionszeit in der Veresterung deutlich verkürzt. Sie kann durch die Verwendung der Schleppmittel mehr als halbiert werden,
wobei gleichzeitig ein quantitativer Umsatz der Säuren zum Ester erreicht werden kann, während ohne Schleppmittel die Reaktion nur bis zu einem Umsatz von annähernd 85 % Ausbeute laufen.

Der Reaktionsverlauf im erfindungsgemäßen Verfahren läßt sich leicht mit üblichen Methoden beispielsweise mittels HPLC, Dünnschichtchromatographie oder Gaschromatographie verfolgen.

Die Erfindung eignet sich auch zur Extraktion von Verbindungen der Formel II gemäß Anspruch 1 aus wäßrigen Lösungen, dadurch gekennzeichnet, daß die Verbindungen der allgemeinen Formel II direkt oder nach Freisetzung aus ihren Salzen in Gegenwart eines C₁-C₈-Alkohols und einem mit Wasser nicht mischbaren Lösungsmittel extrahiert werden.

Die folgenden Beispiele dienen der Veranschaulichung der Erfindung.

Bei der Enzym-katalysierten kinetischen Racematspaltung von Aminen, wie sie in WO 95/08636 beschrieben wird, wird ein Enantiomer in ein Methoxyacetamid (Amid) umgewandelt, dessen Spaltung mit Natronlauge neben dem gewünschten freien Amin auch Natriummethoxyacetat (= NAMA) liefert (siehe Schema I). Die darin gebundene Methoxyessigsäure (= MES) wurde im erfindungsgemäßen Verfahren aus ihrem Salz NAMA freigesetzt, die dann in Wasser gelöste MES wurde anschließend durch Extraktion isoliert und schließlich zum iso-Propylester (= MEIPE), der in der Enzym-katalysierten Racematspaltung als Acylierungsreagenz danach wieder Anwendung findet, verestert. 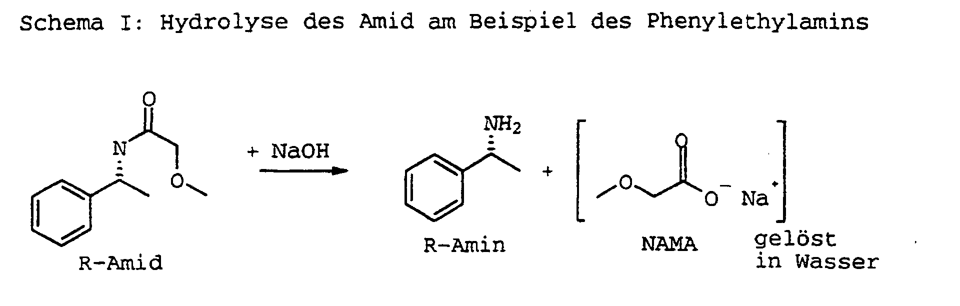 Kernpunkte des erfindungsgemäßen Verfahrens sind die Verwendung von iso-Propanol als Co-Solvens bei der Extraktion der MES aus Wasser, sowie die Zugabe von Cyclohexan als Co-Schlepper bei der Azeotrop-Veresterung des Extraktes.

### Beispiel 1a: Freisetzung der Säure aus dem Salz

Aus 320 g einer wässrigen Lösung von Natriummethoxyacetat (NAMA), die aus einer Amidspaltung mit NaOH erhalten wurde, wurde die Säure freigesetzt (siehe Schema II). Das Amid war aus einer Racematspaltung, die entsprechend dem in WO 95/08636 beschriebenen Verfahren durchgeführt worden war, gewonnen worden.

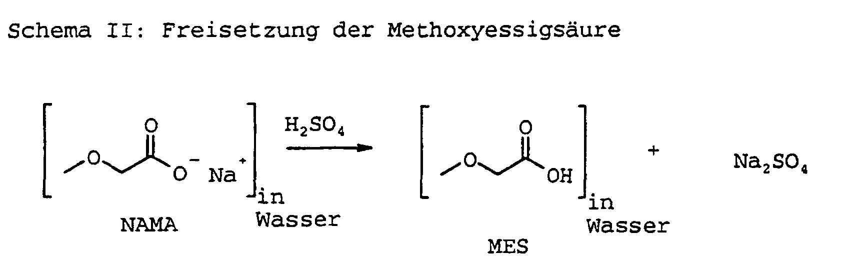

### Zusammensetzung:

| | |
|---|---|
| ca. 3 % | Natronlauge |
| 37 % | Natriummethoxyacetat (NAMA) |

### Durchführung:

Die wässrige Lösung wurde bei Raumtemperatur (ca. 23°C) vorgelegt, und mit der oben angegebenen Schwefelsäure versetzt. Dabei stieg die Temperatur auf 65°C und Natriumsulfat fiel aus. Man kühlte unter Rühren auf Raumtemperatur ab und verdünnte mit 175 ml Wasser, die so erhaltene Lösung wies einen pH-Wert von 1 auf.

Die zugegebene Wassermenge reichte aus, um das entstandene Natriumsulfat bei Raumtemperatur (>22°C) in Lösung zu halten.

Die resultierende Lösung hatte laut ¹H-NMR und Elementaranalytik einen MES-Gehalt von ca. 15 % und einen Na₂SO₄-Gehalt von 17 %.

Die nach dieser Vorschrift erhaltene Lösungen wurde, wenn nicht anders beschrieben, in den folgenden Extraktionsversuchen eingesetzt.

### Beispiel 1b: Extraktion der Säure aus wäßriger Lösung

Da die MES mit Wasser unbegrenzt mischbar ist, konnte sie nicht, wie die Versuche zeigten, mit befriedigendem Erfolg mit unpolaren Lösungsmitteln wie Toluol, Cyclohexan oder Ethern aus dem Wasser isoliert werden. Aufgrund des in diesen Mischungen niedrigen Verteilungskoeffizienten betrug die Extraktionsausbeute < 5 %.

Durch Zugabe eines Alkohols wie iso-Propanol konnte die Extraktion nach Freisetzung der Säure aus ihrem Salz durchgeführt werden.

### a) Extraktion mit verschiedenen Extraktionsmittel-Gemischen:

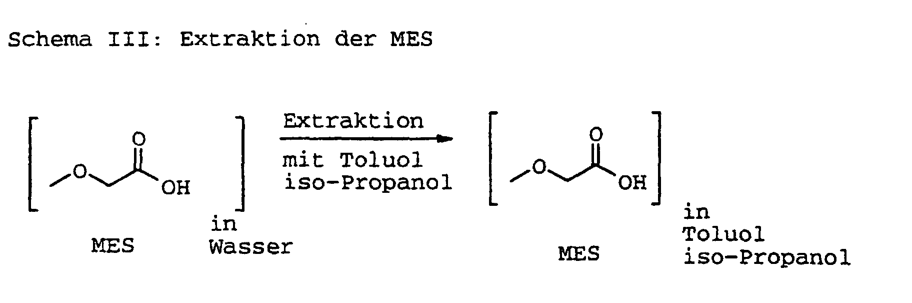

### Einsatzstoffe:

Je 40 g einer wässrigen Lösung von Methoxyessigsäure (MES) mit der folgenden Zusammensetzung:

| | |
|---|---|
| 18,5 % | Methoxyessigsäure (MES) |
| ca. 18 % | Natriumsulfat |

je 40 g Extraktionsmittelgemisch mit der in Tabelle I beschriebenen Zusammensetzung.

**Tabelle I:**

| Zusammensetzung der Extraktionsmittelgemische | | |
|---|---|---|
| Extraktionsmischung | Zusammensetzung [w/w] | |
| | Toluol | iso-Propanol |
| A | 100 | 0 |
| B | 30 | 10 |
| C | 20 | 10 |
| D | 50 | 50 |
| E | 10 | 20 |
| F | 10 | 30 |
| G | 0 | 100 |
| | Cyclohexan | iso-Propanol |
| H | 50 | 50 |

### Durchführung:

Die wässrige Lösung der Methoxyessigsäure wurde bei Raumtemperatur (ca. 23°C) mit den in Tabelle I genannten Extraktionsmittelgemischen versetzt und 30 Minuten auf einem Magnetrührer mit 500 rpm gerührt. Die Mischungen wurden dann jeweils in einen Scheidetrichter überführt und bis zur vollständigen Phasentrennung (ca. 30 Minuten) stehengelassen. Bei den Ansätzen (E, F, H) bei denen Natriumsulfat ausgefallen war, wurde vor der Phasentrennung das Salz durch Erwärmen mit einem Heizluftgebläse (auf ca. 30°C) wieder in Lösung gebracht. Bei Ansatz H bildeten sich drei Phasen aus.

Eine Extraktionsausbeute von 95 % wurde bei Ansatz F erzielt. D und E erbrachten Extraktionsausbeuten von jeweils > 85 % bzw. > 90 %.

### B) Variation des Extraktionsmittel-Feed-Verhältnisses:

### Einsatzstoffe:

wässrige Lösung von Methoxyessigsäure (MES) mit der folgenden Zusammensetzung:

| | |
|---|---|
| 18,5 % | Methoxyessigsäure (MES) |
| ca. 18 % | Natriumsulfat |

= feed
verschiedene Mengen Extraktionsmittelgemisch Toluol / iso-Propanol = 1:1 (w/w)
= Extraktionsmittel

| Extraktion | Feed [g] | Extraktionsmittel [g] | Verhältnis Extraktionsmittel / feed (w/w) |
|---|---|---|---|
| A | 100 | 52,5 | 0,525 |
| B | 50 | 75,0 | 1, 5 |
| C | 50 | 100, 0 | 2,0 |
| D | 50 | 125.0 | 2,5 |
| E | 50 | 1500,0 | 3,0 |

### Durchführung:

Die wässrige Lösung der Methoxyessigsäure wurde bei Raumtemperatur (ca. 23°C) mit den in Tabelle II angegebenen Extraktionsmittelgemischen versetzt und 30 Minuten auf einem Magnetrührer mit 500 rpm gerührt. Die Mischungen wurden dann jeweils in einen Scheidetrichter überführt und bis zur vollständigen Phasentrennung (ca. 30 Minuten) stehengelassen. Bei den Ansätzen, bei denen Natriumsulfat ausgefallen war, wurde vor der Phasentrennung das Salz durch Erwärmen mit einem Heizluftgebläse (auf ca. 30°C) wieder in Lösung gebracht.

Extraktionsausbeuten von > 95 % werden mit Verhältnis > 2 erzielt.

### c) Kontinuierliche Extraktion:

### Einsatzstoffe:

Wässrige Lösung von Methoxyessigsäure (MES) mit der folgenden Zusammensetzung:

| | |
|---|---|
| 17,4 % | Methoxyessigsäure (MES) |
| 17,5 % | Natriumsulfat |

Extraktionsmittel Toluol/iso-Propanol 1:1 (w/w)

### Durchführung:

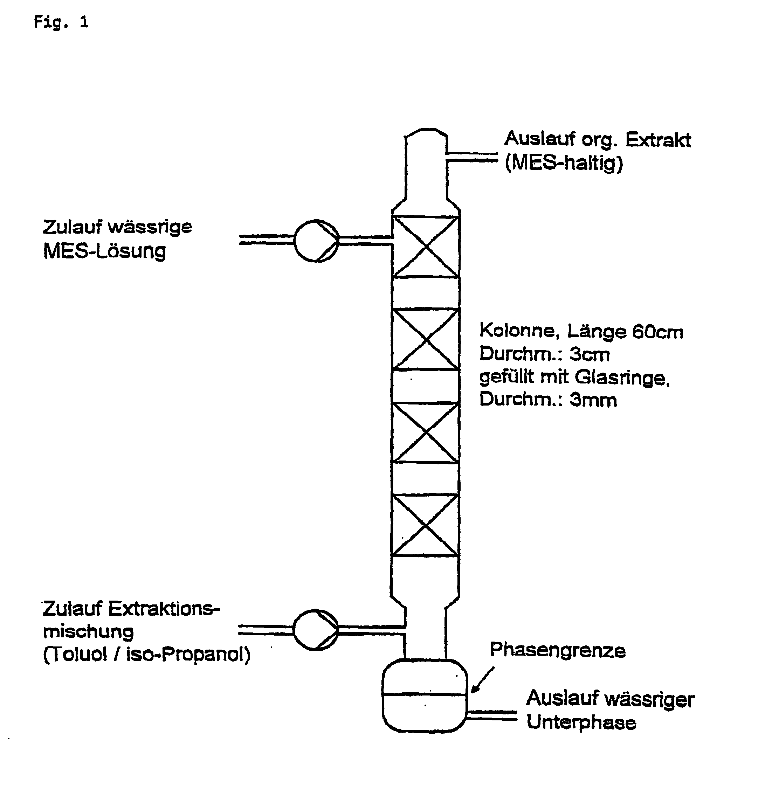

Die wässrige Lösung der Methoxyessigsäure wurde bei Raumtemperatur (ca. 23°C) mit 5 Gew.-% Wasser verdünnt und mit 210 g /Std. auf den Kopf einer Extraktionskolonne (60 cm lang, ⌀ 3 cm, gefüllt mit Glasringen ⌀ 3 mm) gegeben. Im Gegenstrom wurden 200 g /Std. einer Toluol/iso-Propanol-Mischung am Auslauf der Extraktionskolonne zugeführt. Während der Extraktion wurde die Kolonne mit einem Heizband auf ca. 30°C temperiert. Die Phasengrenze wurde so eingestellt, daß sie in der Mitte des am Boden der Kolonne angebrachten Kolbens blieb (siehe Figur 1). Nach 8 Stunden wurde folgendes erhalten:

| | | | |
|---|---|---|---|
| Wässrige Unterphase | 1215 g, | Dichte | 1,24 gcm⁻³ |
| Organischer Extrakt | 2062 g, | Dichte | 0,87 gcm⁻³ |

Der Extrakt (Oberphase) enthielt 12,3 Gew.-% MES.
Die Extraktionsausbeute betrug 91 %.

### Beispiel 3: Veresterung der Säure-haltigen Extrakte am Beispiel der Methoxyessigsäure

Bei der unter Beispiel 2 beschriebenen Extraktion von Methoxyessigsäure (MES) mit Toluol/iso-Propanol fiel ein Extrakt an, der neben dem gewünschten Wertprodukt noch größere Mengen an Wasser enthält.

Wie bei allen H+-katalysierten Veresterungen von freien Carbonsäuren mit Alkoholen, läuft die vorgesehene Veresterung der Methoxyessigsäure mit iso-Propanol in ein Gleichgewicht.

### a) Ionenaustauscher-Katalyse, in Suspension:

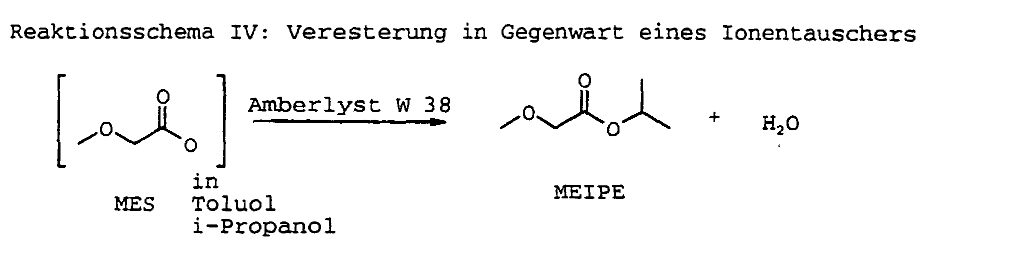

### Einsatzmengen:

4,4 kg Extrakt aus kontinuierlicher Extraktion einer wässrigen MES-Lösung mit folgender Zusammensetzung:

| | |
|---|---|
| 12,3 % | Methoxyessigsäure (MES) |
| 38,1 % | iso-Propanol |
| 38,8 % | Toluol |
| 10,7 % | Wasser |
| 0,1 % | Na₂SO₄ |

15 g saurer Ionenaustauscher Amberlyst W 38

### Durchführung:

Der Extrakt wurde bei Raumtemperatur mit dem sauren Ionenaustauscher versetzt und unter Rühren zum Rückfluß erhitzt. Über eine 20 cm lange mit Drahtfüllkörpern gefüllte Vigreux-Kolonne wurde bei einer Blasentemperatur von 77 bis 79°C ein ternäres Heteroazeotrop, bestehend aus Toluol/i-Propanol und Wasser (Kopftemperatur 76°C) abdestilliert. Dieses Azeotrop wurde über einen Phasenscheider geführt und die untere wäßrige Phase ausgeschleust. Die obere organische Phase wurde in die Kolonne zurückgeführt. Nach 35 Stunden bildete sich im Phasenscheider keine untere Phase mehr aus. Der Wassergehalt in der Blase lag bei 2,2 % (nach Karl-Fischer-Titration). Laut ¹H-NMR-Spektrum war die Methoxyessigsäure zu 85 % umgesetzt. Der Ionentauscher wurde abfiltriert, der Phasenscheider gegen einen Kolonnenkopf ausgetauscht und der Blaseninhalt fraktioniert destilliert.
Isol. Ausbeute MEIPE: 715,5 g (90 % d. Th.)
Reinheit des MEIPE: chem. Reinheit: >99,8 %

### b) Ionenaustauscher-Katalyse, im bypass:

### Einsatzmengen:

785 g Extrakt aus kontinuierlicher Extraktion einer wässrigen MES-Lösung folgender Zusammensetzung:

| | |
|---|---|
| 12,3 % | Methoxyessigsäure (MES) |
| 38,1 % | iso-Propanol |
| 38,8 % | Toluol |
| 10,7 % | Wasser |
| 0,1 % | Na₂SO₄ |

50 g saurer Ionenaustauscher Amberlyst W 38

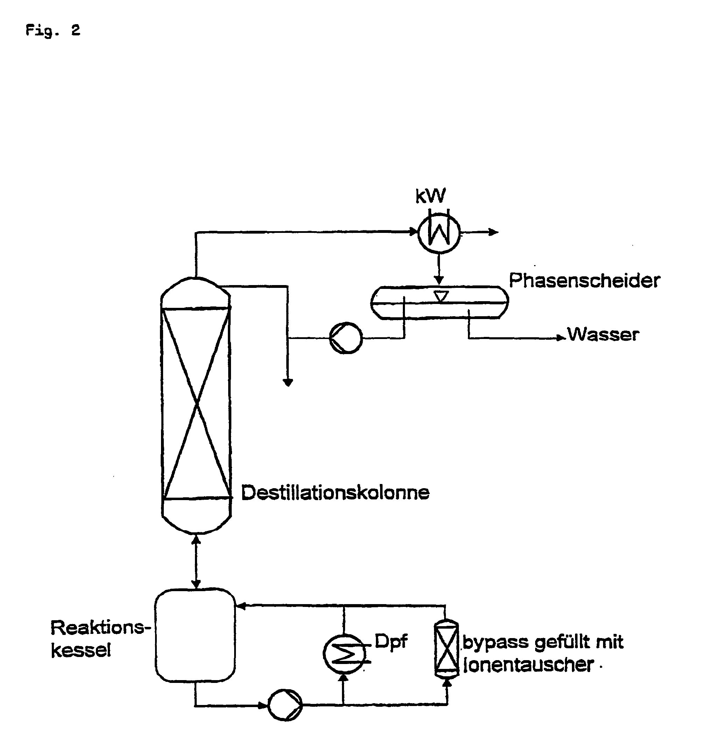

Die Apparatur ist Figur 2 zu entnehmen.

### Durchführung:

Der Extrakt wurde bei Raumtemperatur in ein 1 1-Miniplant-Gefäß gegeben. Der Bodenauslauf des Gefäßes wurde geöffnet und das Gemisch mit einer Membranpumpe (Leistung: 4,8 1/Std.) über eine mit 50 g saurem Ionentauscher Amberlyst W 38 gefüllte Säule (Länge 30 cm, ⌀: 3 cm) gepumpt. Die Beheizung des Miniplant-Gefäßes wurde auf 140°C genommen und das entstehende Destillat über eine 20 cm lange Vigreux-Kolonne (mit Drahtfüllkörpern gefüllt) bei einer Blasentemperatur von 78 bis 80°C abdestilliert. Das gebildete ternäre Heteroazeotrop, bestehend aus Toluol/ i-Propanol und Wasser (Kopftemperatur 76°C) wurde über einen Phasenscheider geführt und die untere wäßrige Phase ausgeschleust. Die obere organische Phase wurde in die Kolonne zurückgeführt. Um Temperaturverluste zu vermeiden, wurde auch der mit Ionentauscher gefüllte bypass mit einem Heizband beheizt. Beim Austritt aus dem bypass wies das Reaktionsgemisch die gleiche Temperatur wie im Miniplant-Gefäß auf. Nach 10 Stunden bildete sich im Phasenscheider keine untere Phase mehr aus.

Das Gemisch wurde fraktioniert desilliert.

Isol. Ausbeute MEIPE: 82 g (56 % d. Th.)

Die geringe Ausbeute ist z.T. darauf zurückzuführen, daß ein Teil des Wertproduktes im mit Ionentauscher gefüllten bypass zurückblieb.

Reinheit des MEIPE: chem. Reinheit: 98,5 % (enthält Toluol)

### c) H₂SO₄-Katalyse:

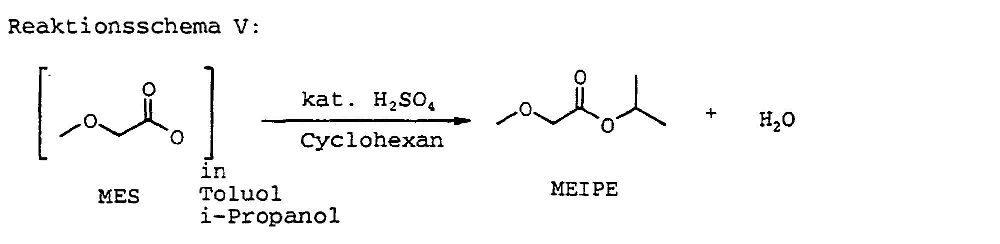

### Einsatzmengen:

1 kg Extrakt aus kontinuierlicher Extraktion einer wässrigen MES-Lösung folgender Zusammensetzung:

| | |
|---|---|
| 10,7 % | Methoxyessigsäure (MES) |
| 2,0 % | Methoxyessigsäure-iso-Propylester |
| 36,0 % | iso-Propanol |
| 38,0 % | Toluol |
| 14,0 % | Wasser |
| 0, 04 % | Na₂SO₄ |

### Zugegeben:

| | |
|---|---|
| 40,0 g | Methoxyessigsäure (MES) |
| 0,8 g | konz. H₂SO₄ (0,5 mol-%) |
| 100 ml | Cyclohexan |

### Durchführung:

Der Extrakt wurde bei Raumtemperatur (ca. 23°C) mit der Methoxyessigsäure, Schwefelsäure und Cyclohexan vermischt und anschließend zum Rückfluß erhitzt. Über eine 30 cm lange, mit Drahtfüllkörpern gefüllte Kolonne wurde bei einer Blasentemperatur von 74 bis 76°C ein ternäres Heteroazeotrop, bestehend aus Cyclohexan / i-Propanol und Wasser (Kopftemperatur 66°C) abdestilliert. Dieses Azeotrop wurde über einen zuvor mit Cyclohexan gefüllten Phasenscheider geführt und die untere wäßrige Phase ausgeschleust. Die obere organische Phase wurde in die Kolonne zurückgeführt. Nach 16 Stunden war die Innentemperatur auf 92°C angestiegen, die Kopftemperatur in der Kolonne betrug 70°C und der Wassergehalt in der Blase lag bei 0,07 % (nach Karl-Fischer-Titration). Laut ¹H-NMR-Spektrum war die Methoxyessigsäure zu >98 % umgesetzt.

Der Blaseninhalt wurde fraktioniert destilliert.
Isol. (= isolierte) Ausbeute MEIPE: 220 g (94 % d.Th. = der Theorie)
Reinheit des MEIPE: chem. (= chemische) Reinheit > 99,8 %

### d) Azeotrop-Veresterung von Methoxyessigsäure/H₂SO₄-Katalyse:

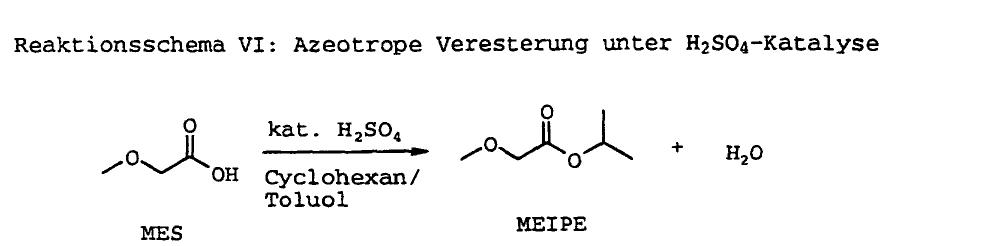

### Einsatzmengen:

| | |
|---|---|
| 400 g | (4,44 mol) Methoxyessigsäure |
| 400 g | Cyclohexan |
| 300 g | Toluol |
| 600 g | (10 mol) iso-Propanol |
| 1 g | (0,01 mol) konz. Schwefelsäure (entspricht 0,2 mol-%) |

### Durchführung:

Bei Raumtemperatur wurden Methoxyessigsäure, Cyclohexan, Toluol, iso-Propanol und Schwefelsäure vorgelegt und anschließend zum Rückfluß erhitzt. Über eine 30 cm lange, mit Drahtfüllkörpern gefüllte Kolonne wurde bei einer Blasentemperatur von 74 bis 76°C ein ternäres Heteroazeotrop, bestehend aus Cyclohexan/i-Propanol und Wasser (Kopftemperatur 66°C) abdestilliert. Dieses Azeotrop wurde über einen zuvor mit Cyclohexan gefüllten Phasenscheider (Inhalt: ca. 600 ml) geführt. Die untere, wäßrige Phase verdrängte dabei die entsprechende Menge Cyclohexan, das über die Kolonne in den Reaktionsansatz zurückfloß. Nach 6 Stunden waren laut ¹H-NMR 75 % der Methoxyessigsäure umgesetzt, der Wassergehalt in der Destillationsblase lag bei 0,58 %. Nach 16 Stunden bildete sich im Phasenscheider kein Wasser mehr aus; die Kopftemperatur in der Kolonne war auf 70°C angestiegen und der Wassergehalt in der Blase lag bei 0,01 % (nach Karl-Fischer-Titration). Laut ¹H-NMR-Spektrum war die Methoxyessigsäure quantitativ umgesetzt worden.

Der Phasenscheider wurde gegen einen Kolonnenkopf ausgetauscht und der Blaseninhalt fraktioniert destilliert.
Isol. Ausbeute MEIPE: 562 g (97 % d. Th.)
Reinheit des MEIPE: chem. Reinheit: >99,8 %

## Patentansprüche

1. Verfahren zur Herstellung von Estern der allgemeinen Formel I, 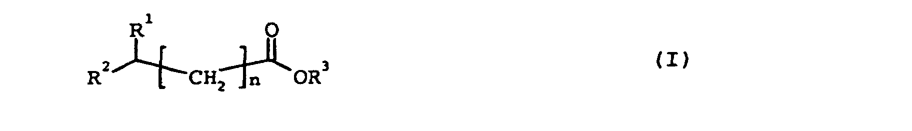 aus in wäßrigen Lösungen enthaltenen Verbindungen der allgemeinen Formel II, 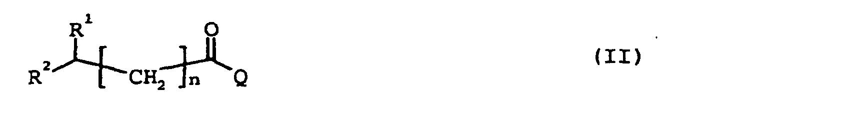 **dadurch gekennzeichnet, daß**
a) die Verbindungen der allgemeinen Formel II direkt oder nach Freisetzung aus ihren Salzen in Gegenwart eines C₁-C₈-Alkohols und einem mit Wasser nicht mischbaren Lösungsmittel, wobei der Alkohol auch gleichzeitig als Lösungsmittel verwendet werden kann, extrahiert werden und
b) anschließend in Gegenwart eines Katalysators und eines Schleppmittels, dessen Siedepunkt als Azeotrop mit dem. Alkohol und dem Wasser um mindestens 8°C niedriger ist als das Azeotrop aus dem Alkohol und dem Wasser, unter Bedingungen einer azeotropen Destillation mit dem C₁-C₈-Alkohol verestert werden, wobei mindestens 1 Gew-% Schleppmittel bezogen auf das Gesamtvolumen der Reaktion verwendet werden und
wobei die Verfahrensschritte (a) und (b) zeitlich und räumlich getrennt oder aber in einer aufeinanderfolgenden kontinuierlichen oder diskontinuierlichen Sequenz durchgeführt werden können und wobei die Variablen und Substituenten in den Formeln I und II folgende Bedeutung haben:
R¹ = F, Cl, -OH, substituiertes oder unsubstituiertes, verzweigtes oder unverzweigtes -OC₁-C₁₀-Alkyl,
R² = H, substituiertes oder unsubstituiertes, verzweigtes oder unverzweigtes C₁-C₁₀-Alkyl
R³ = substituiertes oder unsubstituiertes, verzweigtes oder unverzweigtes C₁-C₈-Alkyl,
Q = -OH, -O⁻ K⁺, wobei K⁺ ein Alkali- oder Erdalkalikation oder ein Amin ist,
n = 0,1 oder 2.

2. Verfahren nach Anspruch 1, **dadurch gekennzeichnet, daß** zur Freisetzung der Säure aus den Salzen der Verbindungen der Formel II eine Säure verwendet wird, deren pKs-Wert niedriger ist als der der Verbindungen der Formel II.

3. Verfahren nach Anspruch 1 oder 2, **dadurch gekennzeichnet, daß** zur Freisetzung der Säure aus den Salzen der Verbindungen der Formel II H₂SO₄ verwendet wird.

4. Verfahren nach den Ansprüchen 1 bis 3, **dadurch gekennzeichnet, daß** als Alkohol Ethanol, Propanol, iso-Propanol, Butanol, Hexanol oder Octanol verwendet wird.

5. Verfahren nach den Ansprüchen 1 bis 4, **dadurch gekennzeichnet, daß** der Alkohol gleichzeitig auch als organisches Lösungsmittel verwendet wird.

6. Verfahren nach den Ansprüchen 1 bis 5, **dadurch gekennzeichnet, daß** Verfahrensschritt (a) bei einer Temperatur von 0°C bis 70°C durchgeführt wird.

7. Verfahren nach den Ansprüchen 1 bis 6, **dadurch gekennzeichnet, daß** der C₁-C₈-Alkohol und das mit Wasser nicht mischbare Lösungsmittel getrennt zur Extraktion (a) zugegeben werden.

8. Verfahren nach den Ansprüchen 1 bis 7, **dadurch gekennzeichnet, daß** die Verbindungen der Formel II kontinuierlich extrahiert werden.

9. Verfahren nach den Ansprüchen 1 bis 8, **dadurch gekennzeichnet, daß** in Verfahrensschritt (a) die Säuren der Verbindungen der Formel II mit einer Ausbeute von mindestens 85 % extrahiert werden.

10. Verfahren nach den Ansprüchen 1 bis 9, **dadurch gekennzeichnet, daß** als Lösungsmittel in Verfahrensschritt (a) apolare organische Lösungsmittel verwendet werden.

11. Verfahren nach den Ansprüchen 1 bis 10, **dadurch gekennzeichnet, daß** als Katalysator für die Veresterung in Verfahrensschritt (b) saure Ionenaustauscher oder Mineralsäuren verwendet werden.

12. Verfahren nach den Ansprüchen 1 bis 11, **dadurch gekennzeichnet, daß** als Schleppmittel Cyclohexan verwendet wird.

13. Verfahren nach den Ansprüchen 1 bis 12, **dadurch gekennzeichnet, daß** Verfahrensschritt (b) bei einem Druck größer ein bar durchgeführt wird.

14. Verfahren nach den Ansprüchen 1 bis 13, **dadurch gekennzeichnet, daß** der Wassergehalt während der Veresterung in Verfahrensschritt (b) unter 0,1 % gedrückt wird.

15. Verfahren nach den Ansprüchen 1 bis 14, **dadurch gekennzeichnet, daß** die Ausbeute des Esters der allgemeinen Formel I in Verfahrensschritt (b) mindestens 90 % beträgt.

16. Verfahren nach einem der vorstehenden Ansprüche, **dadurch gekennzeichnet, daß** als Ester C₁-C₈-Alkylester einer C₁-C₄-Alkoxyessigsäure hergestellt werden.

17. Verfahren zur Extraktion von Verbindungen der Formel II gemäß Anspruch 1 aus wässrigen Lösungen, **dadurch gekennzeichnet, daß** die Verbindungen der allgemeinen Formel II direkt oder nach Freisetzung aus ihren Salzen über Ansäuern in Gegenwart eines C₁-C₈-Alkohols und einem mit Wasser nicht mischbaren Lösungsmittel extrahiert werden.

## Claims

1. A process for the preparation of esters of the general formula I 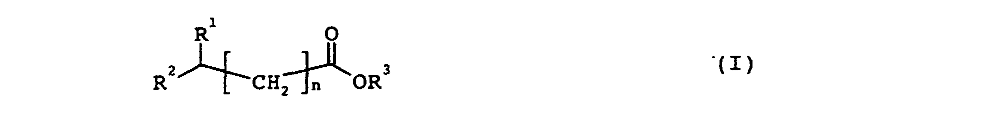 from compounds of the general formula II contained in aqueous solutions 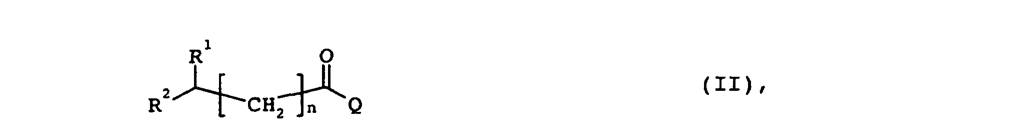 **characterized in that**
a) the compounds of the general formula II are extracted directly or after liberation from their salts in the presence of a C₁-C₈-alcohol and a water-immiscible solvent, wherein the alcohol can also simultaneously be used as a solvent, and
b) then esterifyed with the C₁-C₈-alcohol in the presence of a catalyst and of an entraining agent, whose boiling point as an azeotrope with the alcohol and the water is lower by at least 8°C than that of the azeotrope of the alcohol and the water, under the conditions of an azeotropic distillation, wherein at least 1% by weight of entraining agent is used based on the total volume of the reaction,
wherein the process steps (a) and (b) can be carried out separately in terms of time and space or else in a successive continuous or batchwise sequence and wherein the variables and substituents in the formulae I and II have the following meanings:
R¹ = F, Cl, -OH, substituted or unsubstituted, branched or unbranched -OC₁-C₁₀-alkyl,
R² = H, substituted or unsubstituted, branched or unbranched C₁-C₁₀-alkyl,
R³ = substituted or unsubstituted, branched or unbranched C₁-C₈-alkyl,
Q = -OH, -O⁻ K⁺, where K⁺ is an alkali metal cation or alkaline earth metal cation or an amine,
n = 0,1 or 2.

2. A process as claimed in claim 1, **characterized in that** an acid whose pKₐ is lower than that of the compounds of the formula II is used for the liberation of the acid from the salts of the compounds of the formula II.

3. A process as claimed in claim 1 or 2, **characterized in that** H₂SO₄ is used for the liberation of the acid from the salts of the compounds of the formula II.

4. A process as claimed in any of claims 1 to 3, **characterized in that** the alcohol used is ethanol, propanol, isopropanol, butanol, hexanol or octanol.

5. A process as claimed in any of claims 1 to 4, **characterized in that** the alcohol is simultaneously also used as an organic solvent.

6. A process as claimed in any of claims 1 to 5, **characterized in that** process step (a) is carried out at a temperature of from 0°C to 70°C.

7. A process as claimed in any of claims 1 to 6, **characterized in that** the C₁-C₈-alcohol and the water-immiscible solvent are added separately to the extraction (a).

8. A process as claimed in any of claims 1 to 7, **characterized in that** the compounds of the formula II are extracted continuously.

9. A process as claimed in any of claims 1 to 8, **characterized in that** the acids of the compounds of the formula II are extracted with a yield of at least 85% in process step (a).

10. A process as claimed in any of claims 1 to 9, **characterized in that** the solvents used in process step (a) are apolar organic solvents.

11. A process as claimed in any of claims 1 to 10, **characterized in that** the catalyst used for the esterification in process step (b) is an acidic ion exchanger or mineral acids.

12. A process as claimed in any of claims 1 to 11, **characterized in that** the entraining agent used is cyclohexane.

13. A process as claimed in any of claims 1 to 12, **characterized in that** process step (b) is carried out at a pressure of greater than 1 bar.

14. A process as claimed in any of claims 1 to 13, **characterized in that** the water content during the esterification in process step (b) is lowered below 0.1%.

15. A process as claimed in any of claims 1 to 14, **characterized in that** the yield of the ester of the general formula I in process step (b) is at least 90%.

16. A process as claimed in any of the preceding claims, **characterized in that** the ester prepared is a C₁-C₈-alkyl ester of a C₁-C₄-alkoxyacetic acid.

17. A process for the extraction of compounds of the formula II as claimed in claim 1 from aqueous solutions, **characterized in that** it comprises extracting the compounds of the general formula II directly or after liberation from their salts by means of acidification in the presence of a C₁-C₈-alcohol and a water-immiscible solvent.

## Revendications

1. Procédé de préparation d'esters de la formule générale I : 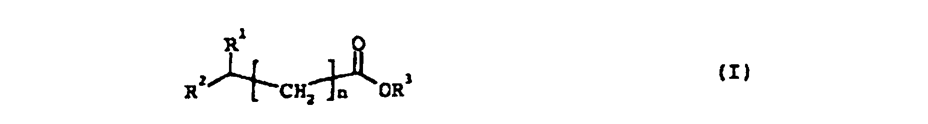 à partir de composés de la formule générale II, présents en solutions aqueuses : 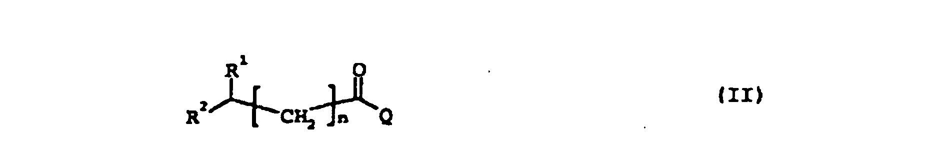 **caractérisé en ce que**
a) les composés de la formule générale II sont extraits directement ou après libération de leur sel, en présence d'un alcool en C₁-C₈ et d'un solvant non miscible à l'eau, où l'alcool peut être utilisé également simultanément comme solvant, et
b) ensuite on estérifie avec l'alcool en C₁-C₈ en présence d'un catalyseur et d'un agent d'entraînement, dont le point d'ébullition est inférieur d'au moins 8°C par rapport à l'azéotrope de l'alcool et de l'eau, dans les conditions d'une distillation azéotropique, où au moins 1% en poids d'agent d'entraînement, sur base du volume total de la réaction, est utilisé, et
où les étapes de procédé (a) et (b) peuvent être réalisées de manière séparée temporellement et spatialement, ou en séquence successive de manière continue ou discontinue et où les variables et substituants dans les formules I et II ont la signification suivante :
R¹ = F, Cl, OH, -O-(alkyle C₁-C₁₀) substitué ou non substitué, linéaire ou ramifié,
R² = H, alkyle C₁-C₁₀ substitué ou non substitué, linéaire ou ramifié,
R³ = alkyle C₁-C₈ substitué ou non substitué, linéaire ou ramifié,
Q = OH, -O⁻K⁺, où K⁺ est un ion alcalin ou alcalino-terreux ou une amine,
n = 0, 1 ou 2.

2. Procédé selon la revendication 1, **caractérisé en ce que** pour libérer l'acide à partir des sels des composés de la formule II, on utilise un acide, dont le pK est inférieur à celui des composés de la formule II.

3. Procédé selon la revendication 1 ou 2, **caractérisé en ce que** pour libérer l'acide à partir des sels des composés de la formule II, on utilise H₂SO₄.

4. Procédé selon les revendications 1 à 3, **caractérisé en ce que** l'on utilise comme alcool, l'éthanol, le propanol, l'isopropanol, le butanol, l'hexanol ou l'octanol.

5. Procédé selon les revendications 1 à 4, **caractérisé en ce que** l'on utilise l'alcool, simultanément au solvant organique.

6. Procédé selon les revendications 1 à 5, **caractérisé en ce que** l'étape de procédé (a) est réalisée à une température allant de 0°C à 70°C.

7. Procédé selon les revendications 1 à 6, **caractérisé en ce que** l'on ajoute l'alcool C₁-C₈ et le solvant non miscible à l'eau, séparément pour l'extraction (a).

8. Procédé selon les revendications 1 à 7, **caractérisé en ce que** les composés de la formule II sont extraits en continu.

9. Procédé selon les revendications 1 à 8, **caractérisé en ce que** dans l'étape de procédé (a), les acides des composés de la formule II sont extraits avec un rendement d'au moins 85%.

10. Procédé selon les revendications 1 à 9, **caractérisé en ce que** l'on utilise comme solvant, dans l'étape de procédé (a), des solvants organiques apolaires.

11. Procédé selon les revendications 1 à 10, **caractérisé en ce que** l'on utilise comme catalyseur pour l'estérification dans l'étape de procédé (b), un échangeur d'ions acide ou des acides minéraux.

12. Procédé selon les revendications 1 à 11, **caractérisé en ce que** l'on utilise comme agent d'entraînement, le cyclohexane.

13. Procédé selon les revendications 1 à 12, **caractérisé en ce que** l'étape de procédé (b) est réalisée à une pression supérieure à un bar.

14. Procédé selon les revendications 1 à 13, **caractérisé en ce que** la teneur en eau pendant l'estérification de l'étape de procédé (b), est maintenue sous 0,1%.

15. Procédé selon les revendications 1 à 14, **caractérisé en ce que** le rendement en ester de la formule générale I se situe à au moins 90% dans l'étape de procédé (b).

16. Procédé selon l'une des revendications précédentes, **caractérisé en ce que** l'on prépare comme ester, un ester d'alkyle C₁-C₈ d'un acide (alcoxy C₁-C₄) acétique.

17. Procédé d'extraction de composés de la formule II selon la revendication 1, depuis des solutions aqueuses, **caractérisé en ce que** les composés de la formule générale II sont extraits directement ou après libération de leurs sels par acidification en présence d'un alcool C₁-C₈ et d'un solvant non miscible à l'eau.
